Europäisches Patentamt

European Patent Office

Office européen des brevets

(19)

(11) Publication number : **0 349 298 B1**

(12) **EUROPEAN PATENT SPECIFICATION**

(45) Date of publication of patent specification :
20.01.93 Bulletin 93/03

(51) Int. Cl.⁵ : **C07C 19/08,** C07C 17/20,
B01J 27/125

(21) Application number : **89306574.8**

(22) Date of filing : **29.06.89**

(54) **Gas-phase hydrofluorination process.**

(30) Priority : **29.06.88 US 212964**

(43) Date of publication of application :
**03.01.90 Bulletin 90/01**

(45) Publication of the grant of the patent :
**20.01.93 Bulletin 93/03**

(84) Designated Contracting States :
**AT BE CH DE ES FR GB GR IT LI LU NL SE**

(56) References cited :
**DE-A- 2 128 341**
**GB-A- 1 000 083**
**US-A- 3 258 500**
**US-A- 3 755 477**

(73) Proprietor : **E.I. DU PONT DE NEMOURS AND
COMPANY
1007 Market Street
Wilmington Delaware 19898 (US)**

(72) Inventor : **Manzer, Leo Ernest
714 Burnley Road
Wilmington Delaware 19803 (US)**
Inventor : **Rao, Velliyur Nott Mallikarjuna
1, Georgetown Avenue
Wilmington Delaware 19809 (US)**

(74) Representative : **Jones, Alan John et al
CARPMAELS & RANSFORD 43 Bloomsbury
Square
London, WC1A 2RA (GB)**

EP 0 349 298 B1

Jouve, 18, rue Saint-Denis, 75001 PARIS

## Description

An improved process for the manufacture of 1,1,1-trifluorodichloroethane and/or 1,1,1,2-tetrafluorochloroethane, more particularly, a gas-phase reaction of selected pentahaloethanes with hydrogen fluoride in the presence of a selected metal on a high fluorine content alumina support, the reaction being conducted under controlled conditions whereby the production of pentafluoroethane is miminized.

## BACKGROUND OF THE INVENTION

GB 1,000,485 describes a process for the preparation of organic fluorinated compounds by fluorination of halo-olefins in the gaseous phase and at a temperature preferably within the range 200°C to 400°C. The catalyst consists essentially of partially fluorinated alumina impregnated with one or more polyvalent metal halides. The polyvalent metal may be chromium, cobalt, nickel, or manganese. The total content of polyvalent metal halide expressed as oxide is not more than 15% by weight of the partially fluorinated (70-80%) alumina expressed as alumina. This patent shows that addition of HF to $CCl_2=CCl_2$ produces $CCl_2FCHCl_2$ which through a series of HCl eliminations and HF additions produces $CF_3CHCl_2$. In addition, the patent states that if fluorination of the catalyst is excessive, the activity of the catalyst is impaired (page 3, column 2, lines 85-87).

GB 1,000,083 describes a process for the preparation of 1,1,1-trifluoro-2-chloroethane by reacting tetrachloroethane and hydrogen fluoride in the gaseous phase at a temperature not above 450°C in the presence of a catalyst based on partially fluorinated alumina, based on alumina activated by impregnation with chromium, cobalt, nickel and manganese. On page 2, column 1 of GB 1,000,083, the fluorination of the alumina is taught via the exposure of alumina to HF "so as to obtain a partial (70%-80%) fluorination of the alumina." It is also noted on page 2 column 1 that "If the amount of fluorination is excessive, the activity of the catalyst is impaired."

U.S. 3,755,477 describes a process for producing fluorinated aliphatic hydrocarbons which comprises fluorinating a halogenated aliphatic hydrocarbon containing at least one halogen atom other than fluorine by reaction in the gas phase with hydrogen fluoride in the presence of a steam-treated and calcined chromium oxide catalyst prepared by a multi-step process. Example 25 on Column 5 of this patent shows the hydrofluorination of $CF_3CHCl_2$ which yields 67% $CF_3CHF_2$, 21% $CF_3CHClF$, and 2.5% $CF_3CClF_2$. This example shows that the less desirable pentafluorinated product is obtained in a greater amount than the desired tetrafluorinated product.

U.S. 3,258,500 describes a process for the catalytic vapor phase reaction of HF with halohydrocarbons and more particularly to the substitution of fluorine for other halogens in halohydrocarbons which contain halogens other than or in addition to fluorine, employing a catalyst that consists essentially of a heat-activated anhydrous chromium (III) oxide which may be supported on alumina. This catalyst is highly active. Examples 13-16 of this patent show fluorination of fully halogenated ethanes, which, like U.S. 3,755,477, produce the less desirable pentafluorinated ethane in high yields.

The above references do not disclose how to produce selectively both 1,1,1-trifluorodichloroethane and/or 1,1,1,2-tetrafluorochloroethane while minimizing the production of pentafluoroethane.

The process of the instant invention achieves the desired high degree of selectivity by minimizing the formation of pentafluoroethane, through catalyst selection and control of the reaction variables as discussed below and illustrated in the Examples.

## SUMMARY OF THE INVENTION

What has been discovered is a process for the preparation of 1,1,1-trifluorodichloroethane and/or 1,1,1,2-tetrafluorochloroethane by fluorination of a.pentahaloethane of the formula $C_2HX_{5-n}F_n$ wherein X is selected from the group consisting of Cl and Br and wherein n = 0 to 3, comprising contacting in the gaseous phase at 250°C to 450°C said pentahaloethane and HF with a catalyst composition comprising at least one metal in an oxidation state greater than zero, said metal selected from the group consisting of chromium, manganese, nickel, rhodium and cobalt, said metal in combination with an aluminum-containing compound consisting essentially of aluminum and fluorine in such proportions that the fluorine content corresponds to on $AlF_3$ content of at least 90% by weight of the catalyst composition exclusive of the metal, calculated as metal fluoride, said $AlF_3$ content being obtained by pretreatment of the unfluorinated catalyst composition with a vaporizable fluorine-containing compound.

## DETAILS OF THE INVENTION

The preferred pentahaloethanes useful in the practice of this invention are $CCl_3CHCl_2$, $CCl_2FCHCl_2$,

$CClF_2CHCl_2$ and $CF_3CHCl_2$. They may be fed into the reactor individually or as mixtures.

By a high fluorine content aluminum-containing compound is meant a composition comprising aluminum and fluorine in such proportions that the fluorine content corresponds to an $AlF_3$ content of at least 90 weight percent of the catalyst composition, exclusive of the metal, calculated as metal fluoride. The remainder of the composition may include alumina or aluminum oxyfluoride.

The catalyst composition can be prepared in any manner known to the art. For example, the catalyst composition can be prepared by fluorination of alumina impregnated with a solution of at least one chromium, manganese, nickel, rhodium or cobalt compound which may be in the form of the oxide, oxyhalide, halide, pseudohalide, nitrate, sulfate or other compound of the metal. The halides include fluorides, chlorides and bromides. The pseudohalides include cyanides, cyanates and thiocyanates. The preferred metals are manganese, nickel and cobalt. The most preferred metal is cobalt.

In addition, the catalyst composition can also be prepared by co-precipitation of the catalytic metal and the aluminum as the hydroxides which are thereafter dried and calcined to form the mixed oxides, a technique well known to the art. The resulting oxide is then pretreated as described herein.

The total content of chromium, manganese, nickel, rhodium or cobalt expressed as metal is not more than 50% by weight of the catalyst composition and preferably not more than 20% by weight of the catalyst composition, and usually at least 0.02% by weight of the catalyst composition. A more preferred range is 0.1 to 10% by weight of the catalyst composition.

The unfluorinated catalyst composition can be fluorinated to the desired fluorine content by treating with a fluorine-containing compound at elevated temperatures, e.g., at about 200° to about 450°C. The pretreatment with a vaporizable fluorine-containing compound such as HF, $SF_4$, $CCl_3F$, $CCl_2F_2$, $CHF_3$ or $CCl_2FCClF_2$ can be done in any convenient manner including in the reactor which is to be used for contacting the pentahaloethane with HF. By vaporizable fluorine-containing compound is meant a fluorine containing compound which, when passed over the catalyst at the indicated conditions, will fluorinate the catalyst to the desired degree.

A suitable catalyst may be prepared, for example, as follows:

A quantity of $Al_2O_3$ is impregnated with a solution, usually aqueous, of a catalytically effective amount of one or more of the metal compounds described above. By catalytically effective amount is meant an amount of the metal which causes the production of the desired compounds in the process of the instant invention. Normally, this amount. expressed as metal, will be between about 0.02 to 50 weight percent of the alumina support, preferably not more than 20 weight percent, and more preferably 0.1 to 10 weight percent. The impregnated $Al_2O_3$ can be dried until essentially all moisture is removed, e.g., for about 18 hours at about 400°C. The dried catalyst is then transferred to the reactor to be used. The temperature is then gradually increased to about 400°C while maintaining a flow of $N_2$ through the reactor to remove any remaining traces of moisture from the catalyst and the reactor. The temperature is then lowered to about 200°C and HF, or other vaporizable fluorine containing compounds such as $SF_4$, $CCl_3F$, $CCl_2F_2$, $CHF_3$ or $CCl_2FCClF_2$, diluted with $N_2$ is passed through the reactor. The $N_2$ can be gradually reduced until only HF, or other vaporizable fluorine containing compounds such as $SF_4$, $CCl_3F$, $CCl_2 F_2$, $CHF_3$ or $CCl_2 FCClF_2$, is being passed through the reactor. At this point the temperature can be increased to about 450°C and held at that temperature to convert the impregnated $Al_2O_3$ to a fluorine content corresponding to at least 90% $AlF_3$ by weight, e.g., for 15 to 300 minutes, depending on the flow of the fluorine containing compound and the catalyst volume.

The reaction of the pentahaloethane with HF in the presence of the catalyst of the instant invention is conducted at 250°C to 450°C, preferably about 300°C to 400°C, with a contact time of about 5 to 100 seconds preferably about 10 to 90 seconds, and most preferably about 15 to 60 seconds.

The molar ratio of HF to the pentahaloethane can range from about 1/1 to 20/1, preferably about 3/1 to 10/1, and most preferably about 4/1 to 7/1.

A key feature of the invention is that through catalyst selection and process control, as described herein, the desired tri- and/or tetrafluoro products can be obtained as the major products at high pentahaloethane conversions, normally between about 30% and about 90%. Preferably, the reaction variables are controlled so as to keep the production of the pentafluoro product below about 10 percent of the products produced. Thus, as illustrated in the examples with pentahaloethanes, the tri- and tetrafluoro products are obtained in high yields while minimizing the production of higher fluorinated products even at high conversions of pentahaloethane.

Underfluorinated intermediates, such as $CHCl_2CCl_2F$ and $CHCl_2CClF_2$ may be recycled or isolated from the product stream if so desired. In addition 1,1,1-trifluorodichloroethane can be recycled to the reactor for the production of additional 1,1,1,2-tetrafluorochloroethane when this is desired.

The reaction of the pentahaloethane with HF may be conducted in any suitable reactor, including fixed and fluidized bed reactors. The reaction vessel should be constructed from materials which are resistant to the corrosive effects of hydrogen fluoride such as "Hastelloy" and "Inconel".

Pressure is not critical. Atmospheric and superatmospheric pressures are the most convenient and are

EP 0 349 298 B1

therefore preferred.

The fluorinated alkanes produced by the invention have utility as blowing agents and refrigerants. They can also be used as starting materials for the preparation of other useful compounds. For example, 1,1,1,2-tetrafluorochloroethane can be used to prepare 1,1,1,2-tetrafluoroethane.

EXAMPLES

In the following illustrative examples, all parts and percentages are by weight and all temperatures are Centigrade. All reactions used commercial HF containing only trace amounts of water.

General Procedure for Fluorination

The reactor (a 0.5 inch (12.7 mm) ID, 12 inch (305 mm) long Inconel pipe) was charged with the amount of catalyst as described in the following examples, and placed in a sand bath. The bath was gradually heated to 400° while nitrogen gas at a flow rate of 50 ml/minute was passed through the reactor to remove traces of water. The temperature was lowered to 200° and HF and nitrogen gas (1:4 molar ratio) were passed through the reactor and the nitrogen flow was decreased with time until neat HF was being passed through the reactor. At this point, the temperature was gradually raised to 450° and maintained there for 15 to 300 minutes.

The initial fluorine content of the catalyst composition can be determined to correspond to an $AlF_3$ content, exclusive of the metal, of at least 90%. This determination is based on the following calculation related to this reaction:

$$Al_2O_3 + 6\,HF \rightarrow 2\,AlF_3 + 3\,H_2O$$

$y$ = weight of unfluorinated catalyst composition which has been dried at a temperature of at least 400°C for at least four hours in a stream of dry nitrogen, air, or other suitable inert medium, minus the weight of metal compound which is in the unfluorinated dried catalyst composition.

$z$ = weight of the fluorinated catalyst composition minus the weight of metal compound calculated as metal fluoride.

Let $x$ = weight of $Al_2O_3$ remaining after fluorination

$y - x$ = weight of reacted $Al_2O_3$

$z - x$ = weight of $AlF_3$ in the fluorinated catalyst composition

$$(y - x)168/102 = z - x$$

Weight of $AlF_3$ as % of dry fluorinated alumina can then be calculated as follows:

$$\frac{(z - x)}{z}\,100\%$$

The temperature was then decreased to the indicated values and, thereafter, pentahaloethane flow was started. The flows of HF and pentahaloethane were adjusted to give the indicated molar ratios and contact times.

The reactor effluent was scrubbed with aqueous potassium hydroxide to remove HCl and HF and sampled on-line with a Hewlett Packard HP 5890 gas chromatograph using a 20 foot (6.1m) long, one-eighth inch (3.2mm) diameter, column containing "Krytox" perfluorinated polyether on an inert support and a helium flow of 35 cc/minute. Gas chromatogaphic conditions were 70° for 3 minutes followed by temperature programming to 180° at a rate of 6°/minute.

EXAMPLES 1-5

The General Procedure for Fluorination was followed using 19.8 g. (30 cc) of $NiCl_2/Al_2O_3$ (2% Ni) as the initial catalyst charge. The contact time for, all reactions was 30 seconds. The results of the reaction of HF with $CF_3CHCl_2$ over the prepared catalyst are given in Table 1.

| Ex. | Temp. | HF/$CF_3CHCl_2$ | FC123[1] | FC124[2] | FC125[3] |
|-----|-------|-----------------|----------|----------|----------|
| 1 | 350°C | 2/1 | 78.3% | 20.5% | 0.5% |
| 2 | 350°C | 4/1 | 80.6% | 18.4% | 0.4% |
| 3 | 375°C | 2/1 | 60.4% | 35.9% | 2.0% |
| 4 | 400°C | 2/1 | 45.6% | 42.6% | 5.6% |
| 5 | 400°C | 4/1 | 38.7% | 51.5% | 7.5% |

1. FC123 = $CF_3CHCl_2$
2. FC124 = $CF_3CHClF$
3. FC125 = $CF_3CHF_2$

EXAMPLE 6

The General Procedure for Fluorination was followed using 21.0 g. (30 cc) of $CoCl_2/Al_2O_3$ (2% Co) as the initial catalyst charge. The contact time was 30 seconds and the ratio of HF to $CCIF_2CHCl_2$ was 4/1. The temperature was 350°C. Under these conditions the conversion of $CCIF_2CHCl_2$ was 97.8% yielding 56.7% $CF_3CHCl_2$, 13.7% $CF_3CHCIF$ and only 1.1% $CF_3CHF_2$.

EXAMPLE 7

The General Procedure for Fluorination was followed using 20.8 g.(30cc) of $CoCl_2/Al_2O_3$ (4% Co) as the initial catalyst charge. The contact time was 30 seconds and the ratio of HF to $CF_3CHCl_2$ was 2/1. The temperature was 350°C. Under these conditions the conversion of $CF_3CHCl_2$ was 45.7% yielding 90.1% $CF_3CHCIF$ and only 6.7% $CF_3CHF_2$.

**Claims**

1.  A process for the preparation of 1,1,1-trifluorodichloroethane and/or 1,1,1,2-tetrafluorochloroethane by fluorination of a pentahaloethane of the formula $C_2HX_{5-n}F_n$, wherein X is selected from Cl and Br and wherein n = 0 to 3, comprising
    contacting in the gaseous phase at 250°C to 450°C said pentahaloethane and HF with a catalyst composition comprising at least one metal in an oxidation state greater than zero,
    said metal selected from chromium, manganese, nickel, rhodium, and cobalt,
    said metal in combination with an aluminum-containing compound consisting essentially of aluminum and fluorine in such proportions that the fluorine content corresponds to an $AlF_3$ content of at least 90% by weight of the catalyst composition exclusive of said metal, calculated as metal fluoride.

2.  The process of Claim 1 wherein said $AlF_3$ content is obtained by pretreatment of unfluorinated catalyst with a vaporizable fluorine-containing compound at elevated temperatures.

3.  The process of Claim 1 or Claim 2 wherein the catalytically effective amount of metal, expressed as the metal is about 0.02 to about 50 weight percent of the catalyst composition.

4.  The process of Claim 1, 2 or 3 wherein said HF is contacted with the pentahaloethane at a molar ratio of about 1/1 to about 20/1, at a temperature of about 300°C to 400°C, and a contact time of about 5 to 100 seconds.

5

5. The process of any one of Claims 1 to 4 wherein the metal is cobalt.

6. The process of any one of Claims 1 to 5 wherein the pentahaloethane is at least one selected from $CCl_3CHCl_2$, $CCl_2FCHCl_2$, $CClF_2,CHCl_2$ and $CF_3CHCl_2$.

**Patentansprüche**

1. Verfahren zur Herstellung von 1,1,1-Trifluordichlorethan und/oder 1,1,1,2-Tetrafluorchlorethan durch Fluorieren von Pentahalogenethan der Formel $C_2HX_{5-n}F_n$, worin X ausgewählt ist aus Cl und Br und worin n = 0 bis 3 ist, umfassend in Kontakt bringen das Pentahalogenethan und HF mit einer Katalysatorzusammensetzung, die mindestens ein Metall in einer Oxidationsstufe größer als Null umfaßt, in der Gasphase bei 250°C bis 450°C,
wobei das Metall ausgewählt ist aus Chrom, Mangan, Nickel, Rhodium und Kobalt,
wobei das Metall in Kombination vorliegt mit einer Aluminium enthaltenden Verbindung bestehend im wesentlichen aus Aluminium und Fluor in solchen Verhältnissen, daß der Fluorgehalt einem $AlF_3$-Gehalt von mindestens 90 Gewichts-% der Katalysatorzusammensetzung, ausschließend das Metall, berechnet als Metallfluorid, entspricht.

2. Verfahren nach Anspruch 1, worin der $AlF_3$-Gehalt erhalten wird durch Vorbehandlung eines unfluorierten Katalysators mit einer verdampfbaren Fluor enthaltenden Verbindung bei erhöhten Temperaturen.

3. Verfahren nach Anspruch 1 oder 2, worin die katalytisch wirksame Menge an Metall, ausgedrückt als das Metall, etwa 0,02 bis etwa 50 Gewichtsprozent der Katalysatorzusammensetzung beträgt.

4. Verfahren nach Anspruch 1,2 oder 3, worin das HF mit dem Pentahalogenethan in einem molaren Verhältnis von etwa 1/1 bis etwa 20/1 bei einer Temperatur von etwa 300°C bis 400°C und einer Kontaktzeit von etwa 5 bis 100 Sekunden in Kontakt gebracht wird.

5. Verfahren nach einem der Ansprüche 1 bis 4, worin das Metall Kobalt ist.

6. Verfahren nach einem der Ansprüche 1 bis 5, worin das Pentahalogenethan mindestens eines ist ausgewählt aus $CCl_3CHCl_2$, $CCl_2FCHCl_2$, $CClF_2CHCl_2$ und $CF_3CHCl_2$.

**Revendications**

1. Un procédé pour la préparation du 1,1,1-trifluorodichloréthane et/ou du 1, 1, 1, 2-tétrafluorochloréthane par fluoration d'un pentahalogénoéthane de la formule $C_2HX_{5-n}F_n$, où X est choisi entre Cl et Br et où n = 0 à 3, comprenant
la mise en contact en phase gazeuse, entre 250°C et 450°C, dudit pentahalogénoéthane et de HF avec une composition de catalyseur comprenant au moins un métal à un degré d'oxydation supérieur à zéro,
ledit métal étant choisi parmi le chrome, le manganèse, le nickel, le rhodium et le cobalt,
ledit métal étant associé à un composé contenant de l'aluminium constitué essentiellement d'aluminium et de fluor en des proportions telles que la teneur en fluor corresponde à une teneur en $AlF_3$ d'au moins 90 % en poids de la composition de catalyseur à l'exclusion dudit métal, calculé en fluorure de métal.

2. Le procédé de la revendication 1, dans lequel ladite teneur en $AlF_3$ est obtenue par un prétraitement à températures élevées d'un catalyseur non fluoré avec un composé fluoré vaporisable.

3. Le procédé de la revendication 1 ou de la revendication 2, dans lequel la quantité catalytiquement active du métal, exprimée en métal, est d'environ 0, 02 à environ 50 pour cent en poids de la composition de catalyseur.

4. Le procédé de la revendication 1, 2 ou 3, dans lequel ledit HF est mis en contact avec le pentahalogénoéthane à un rapport molaire d'environ 1/1 à environ 20/1, à une température d'environ 300°C à 400°C, et pendant un temps de contact d'environ 5 à 100 secondes.

5. Le procédé de l'une quelconque des revendications 1 à 4, dans lequel le métal est le cobalt.

6. Le procédé de l'une quelconque des revendications 1 à 5, dans lequel le pentahalogénoéthane est au moins un pentahalogénoéthane choisi parmi $CCl_3 CHCl_2$, $CCl_2 FCHCl_2$, $CClF_2 CHCl_2$ et $CF_3 CHCl_2$.